# EUROPEAN PATENT SPECIFICATION

(11) **EP 2 069 460 B1**
(45) Date of publication and mention of the grant of the patent: **16.10.2013**
(21) Application number: 07834859.6
(22) Date of filing: 06.09.2007
(51) Int. Cl.: C09K 11/06, C07D 311/78, C07F 5/02, C07F 9/09, G01N 33/58, G01N 33/92

(54) **FLUORESCENT CELL MARKERS**
FLUORESZIERENDE ZELLENMARKER
MARQUEURS DE CELLULES FLUORESCENTS

(30) Priority: 06.09.2006 NZ 54974206; 07.09.2006 NZ 54974006
(43) Date of publication of application: 17.06.2009
(73) Proprietor: KODE Biotech Limited, Auckland (NZ)
(72) Inventor: Korchagina, Elena, Moscow 117218 (RU); Bovin, Nikolai Vladimirovich, Moscow 117437 (RU); Henry, Stephen Micheal, Ellerslie, Auckland (NZ)
(74) Representative: Gowshall, Jonathan Vallance
(86) International application number: PCT/NZ2007/000256
(87) International publication number: WO 2008/030115

(56) References cited:
- WO-A2-01/91805
- WO-A2-03/082903
- US-A1- 2002 049 986
- HENDRICKSON H STEWART ET AL: "Intramolecularly quenched BODIPY-labeled phospholipid analogs in phospholipase A2 and platelet-activating factor acetylhydrolase assays and in vivo fluorescence imaging", ANALYTICAL BIOCHEMISTRY, ACADEMIC PRESS INC, NEW YORK, vol. 276, no. 1, 1 December 1999 (1999-12-01), pages 27-35, XP002186412, ISSN: 0003-2697, DOI: 10.1006/ABIO.1999.4280
- HYEYOUNG A. CHUNG ET AL: "Casual cell surface remodeling using biocompatible lipid-poly(ethylene glycol)( n ): Development of stealth cells and monitoring of cell membrane behavior in serum-supplemented conditions", JOURNAL OF BIOMEDICAL MATERIALS RESEARCH, vol. 70A, no. 2, 1 August 2004 (2004-08-01), pages 179-185, XP55021841, ISSN: 0021-9304, DOI: 10.1002/jbm.a.20117
- MOTTRAM LAURIE F ET AL: "The Pennsylvania Green Fluorophore: A Hybrid of Oregon Green and Tokyo Green for the Construction of Hydrophobic and pH-Insensitive Molecular Probes", ORGANIC LETTERS, AMERICAN CHEMICAL SOCIETY, US, vol. 8, no. 4, 1 January 2006 (2006-01-01) , pages 581-584, XP002483068, ISSN: 1523-7060, DOI: 10.1021/OL052655G
- 'Phosholipids' MOLECULAR PROBES, PRODUCT INFORMATION, [Online] XP008105364 Retrieved from the Internet: <URL:http://www.probes.invitrogen.com/media /pis/mp00057.pdf>
- RZEPECKI P.W. ET AL.: 'Synthesis of Hybrid Lipid Probes: Derivatives of Phosphatidylethanolamin-Extended Phosphatidylinositol 4,5-Bisphosphate (Pea-PIP2)' J. ORG. CHEM. vol. 67, no. 16, 2002, pages 5454 - 5460, XP002377261
- HASELGRUBER T. ET AL.: 'Synthesis and Applications of a New Poly(ethylene glycol) derivative for the crosslinking of amines with thiols' BIOCONJUGATE CHEM. vol. 6, 1995, page 242, 248, XP000505483

## Description

### TECHNICAL FIELD

The invention relates to fluorescent cell markers. In particular, the invention relates to fluorescent cell markers comprising the fluorophore of fluorescein, BODIPY, or one of their derivatives.

### BACKGROUND ART

The compounds fluorescein, BODIPY, and their derivatives comprise fluorophores.

Fluorescein is water soluble. Using fluorescein as a cell marker requires it to be conjugated to a reactive group such as isothiocyanate. The isothiocyanate group of fluorescein isothiocyanate (FITC) is reactive with the amine group of proteins.

FITC is used to label cells by conjugation with surface expressed proteins. The labeled cells may then be sorted by fluorescent-activated cell sorting (FACS).

The fluorophore of BODIPY has advantageous spectral characteristics over the fluorophore of fluorescein. Derivatives of BODIPY are also used in the labelling of cells by conjugation with surface expressed proteins.

H. S. Hendrickson et. al. ["Intermolecular quenched BODIPY-labelled phospholipid analogs in phospholipase A2 and platelet-activating factor acetylhydrolase assays and in vivo fluorescence imaging" , Analytical Biochemistry, vol. 276, p. 27-35, 1 December 1999] show fluorescent cell markers like PBPEC₆DNP, MBPEDNP, BC₁₁DNPC₈PC and their marking of cells.

Marking of cells by conjugation of a fluorophore with surface expressed proteins may affect cell function. Furthermore, mobility of the fluorophore within the two dimensions of the cell membrane is necessarily dependent on the mobility of the conjugated protein.

Alternative methods of marking cells that may avoid affecting cell function and provide for independent mobility of the fluorophore within the two dimensions of the cell membrane are therefore desired.

It is an object of this invention to provide an alternative method of marking cells or at least to provide a useful choice.

### DISCLOSURE OF INVENTION

In a **first** aspect the invention provides a fluorescent cell marker of the structure:

F-S₁-S₂-L

where
F is a fluorophore;
S₁-S₂ is a spacer linking F to L; and
L is a lipid selected from the group consisting of diacyl- and dialkyl-glycerolipids, including glycerophospholipids.

Preferably, F is selected from the group consisting of: fluorophores of fluorescein, Oregon Green, Pennsylvania Green, Tokyo Green, eosin, BODIPY, BODIPY TR, Alexa Fluor 350, Alexa Fluor 405, Alexa Fluor 488, Alexa Fluor 568, Alexa Fluor 594, Texas Red, Lucifer Yellow, tetramethylrhodamine and their derivatives. Most preferably, F is selected from the group consisting of: fluorophores of fluorescein, BODIPY and their derivatives.

The spacer (S₁-S₂) is selected to provide a water soluble cell marker.

Preferably, the structure includes the substructure: 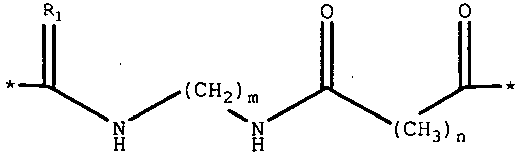 where m and n are independently 3 to 6, R₁ is O or S, and * is other than H. More preferably, the sum of m and n is 6 to 9 and * is C or N.

Preferably where F is the fluorophore of fluorescein or one of its derivatives, S₁ is a C₃₋₅-diaminoalkyl derivative selected from the group consisting of: 1,3-diaminopropyl, 1,4-diaminobutyl, or 1,5-aminopentyl derivatives. More preferably where F is the fluorophore of fluorescein or one of its derivatives, S₁ is a C₃₋₅-aminoalkylthioureidyl. Most preferably where F is the fluorophore of fluorescein or one of its derivatives, S₁ is 5-((5-aminopentyl) thioureidyl.

Preferably where F is the fluorophore of fluorescein or one of its derivatives, S₂ is selected from the group including: - CO(CH₂)₃CO-, -CO(CH₂)₄CO- (adipate), -CO(CH₂)₅CO- and - CO(CH₂)₅NHCO(CH₂)₅CO-. More preferably where F is the fluorophore of fluorescein or one of its derivatives, S₂ is - CO(CH₂)₄CO- (adipate).

Preferably where F is the fluorophore of fluorescein or one of its derivatives the structure includes the substructure: 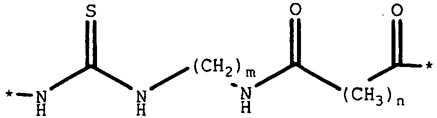 or 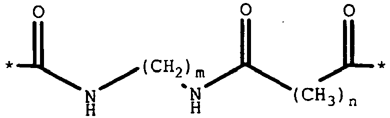 where m and n are independently 3 to 5 and * is other than H.

Preferably where F is the fluorophore of BODIPY or one of its derivatives, S₁ is a C₃₋₅-alkionyldiamine. More preferably where F is the fluorophore of BODIPY or one of its derivatives, S₁ is propionyl ethyldiamine.

Preferably where F is the fluorophore of BODIPY or one of its derivatives, S₂ is selected from the group consiting of: - CO(CH₂)₃CO-, -CO(CH₂)₄CO- (adipate) and -CO(CH₂)₅CO-. More preferably where F is the fluorophore of BODIPY or one of its derivatives, S₂ is -CO(CH₂)₄CO- (adipate).

Preferably where F is the fluorophore of BODIPY or one of its derivatives the structure includes the substructure: 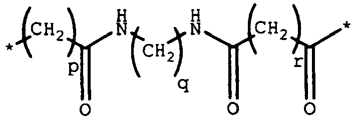 where p, q and r are independently 3 to 5 and * is other than H. More preferably, the sum of p, q and r is 8. Most preferably, p is 2, q is 2 and r is 4.

Preferably L is a lipid selected from the group consisting of diacyl- and dialkyl-glycerolipids, including glycerophospholipids. More preferably L is selected from the group consisting of: diacylglycerolipids, phosphatidate, phosphatidyl choline, phosphatidyl ethanolamine, phosphatidyl serine, phosphatidyl inositol, phosphatidyl glycerol, and diphosphatidyl glycerol derived from one or more of *trans*-3-hexadecenoic acid, *cis*-5-hexadecenoic acid, *cis*-7-hexadecenoic acid, *cis*-9-hexadecenoic acid, *cis*-6-octadecenoic acid, *cis*-9-octadecenoic acid, *trans*-9-octadecenoic acid, *trans*-11-octadecenoic acid, *cis*-11-octadecenoic acid, *cis*-11-eicosenoic acid or *cis*-13-docsenoic acid. More preferably the lipid is derived from one or more cis-desaturated fatty acids. Most preferably L is selected from the group consisting of: 1,2-O-dioleoyl-sn-glycero-3-phosphatidylethanolamine (DOPE), 1,2-O-distearyl-sn-glycero-3-phosphatidylethanolamine (DSPE) and *rac*-1,2-dioleoylglycerol (DOG).

In a **first** embodiment of the first aspect the invention provides a cell marker with the structure: 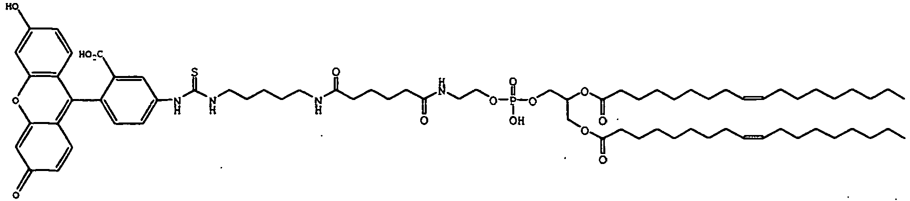 and designated KODE-fluorescein (**I**).

In a **second** embodiment of the first aspect the invention provides a cell marker with the structure: 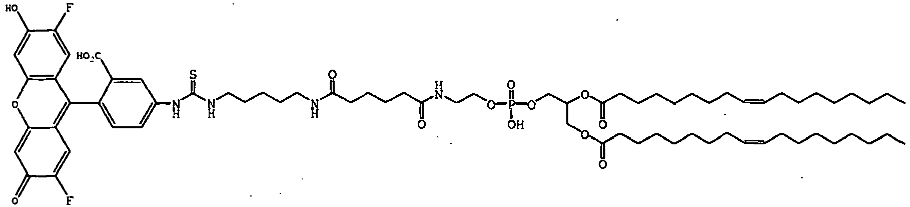 and designated KODE-Oregon Green (**II**).

In a **third** embodiment of the first aspect the invention provides a cell marker with the structure: 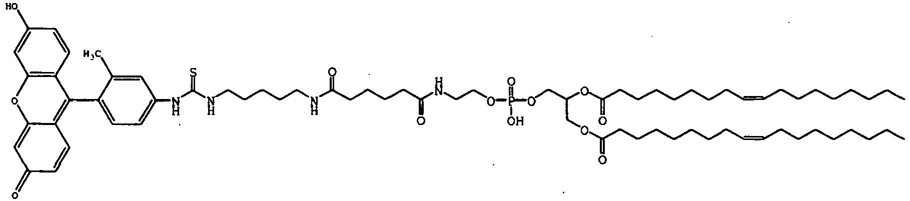 and designated KODE-Tokyo Green (**III**).

In a **fourth** embodiment of the first aspect the invention provides a cell marker with the structure: 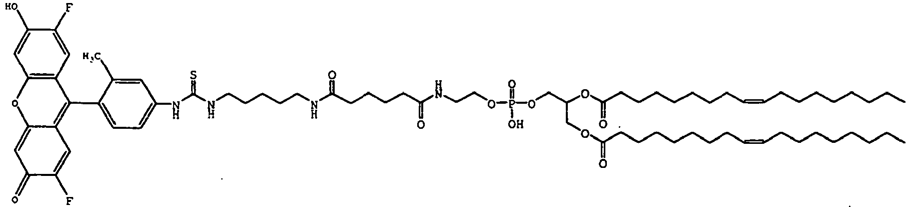 and designated KODE-Pennsylvania Green (**IV**).

In a **fifth** embodiment of the first aspect the invention provides a cell marker with the structure: 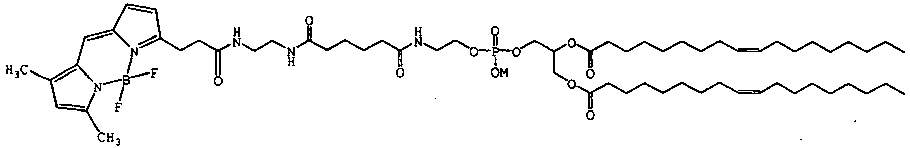 and designated KODE-BODIPY (**V**).

M is typically H, but may be replaced by another monovalent cation such as Na⁺, K⁺ or NH₄⁺.

In a **second** aspect the invention provides a method of marking cells including the step of:
- Contacting a suspension of cells with a cell marker of the first aspect of the invention.

In a **third** aspect the invention provides a cell incorporating a cell marker of the first aspect of the invention.

In a **fourth** aspect the invention provides a cell produced by the method of the second aspect of the invention.

In the context of the description and claims:
"BODIPY" means the compound assigned the Chemical Abstracts Service (CAS) Registry number 138026-71-8 and the CA index name: Boron, difluoro[2-[(2H-pyrrol-2-ylidene-κN)methyl]-1H-pyrrolato-κN]-, (T-4)- (9CI).
"Fluorescein" means the chemical structure assigned the Chemical Abstracts Service (CAS) Registry number 518-47-8 and the CA index name: Spiro[isobenzofuran-1(3H),9'-[9H]xanthen]-3-one, 3',6'-dihydroxy-, sodium salt (1:2).
"Fluorophore" means the substructure or portion of a fluorescent molecule to which the fluorescent properties of the molecule are attributed.
"Or one of its derivatives" means a chemical modification of the chemical structure to provide a fluorophore with substantially equivalent physico-chemical properties, but modified spectral characteristics.
"Water soluble" means a stable, single phase system is formed when the cell marker is contacted with water or saline (such as PBS) in the absence of organic solvents or detergents, and the term "solution" has a corresponding meaning.

Exemplary embodiments of the invention will now be described with reference to the Figures of the accompanying drawings pages.

### BRIEF DESCRIPTION OF FIGURES

**Figure 1****.** Red blood cells following contact with cell marker (I) viewed with a fluorescence microscope at 470nm under 250x magnification.

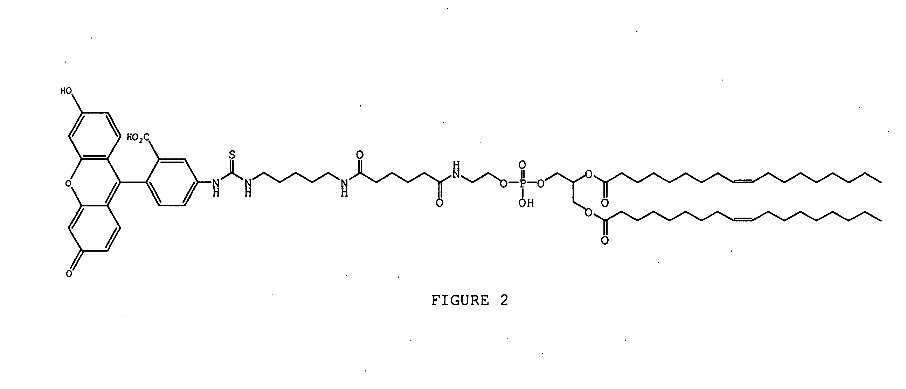

**Figure 2****.** Structure of cell marker designated KODE-fluorescein (I).

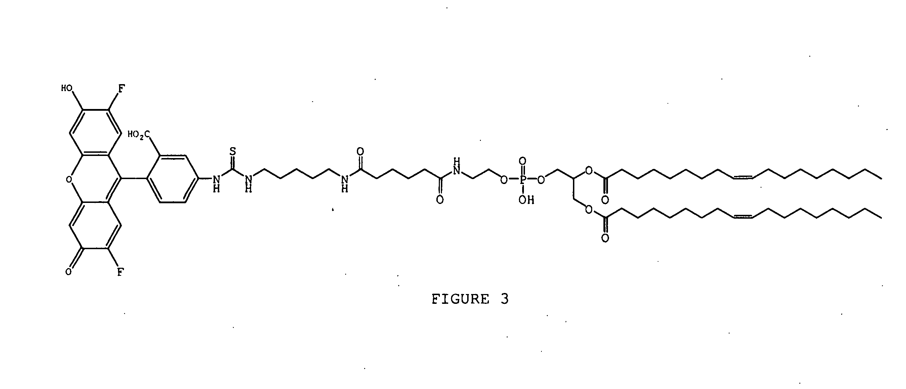

**Figure 3****.** Structure of cell marker designated KODE-Oregon Green (II).

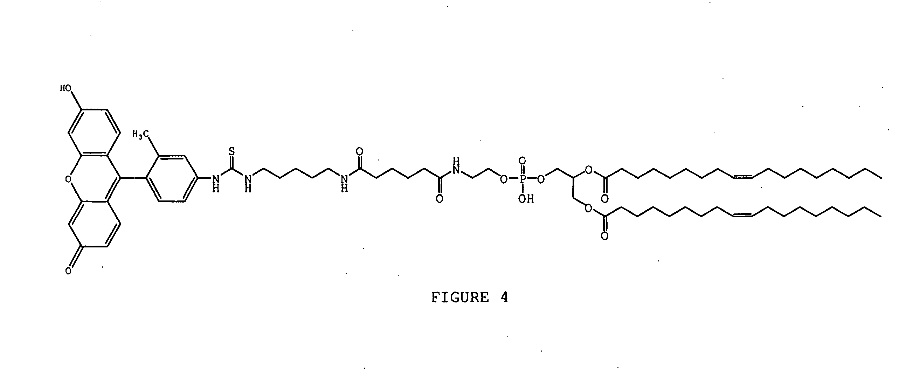

**Figure 4****.** Structure of cell marker designated KODE-Tokyo Green (III).

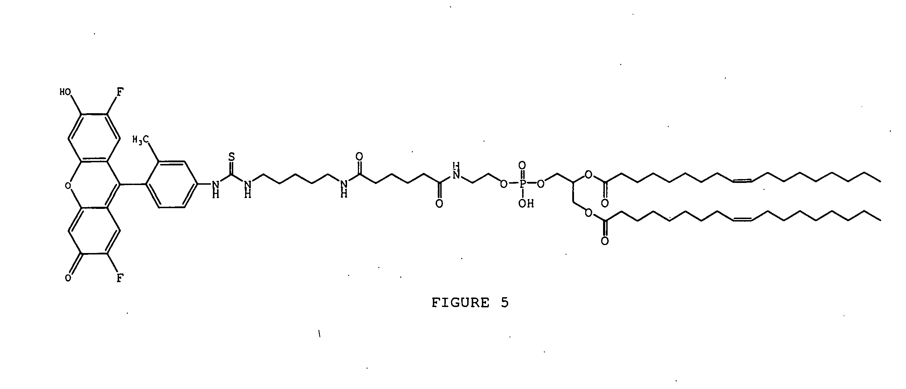

**Figure 5****.** Structure of cell marker designated KODE-Pennsylvania Green (**IV**).

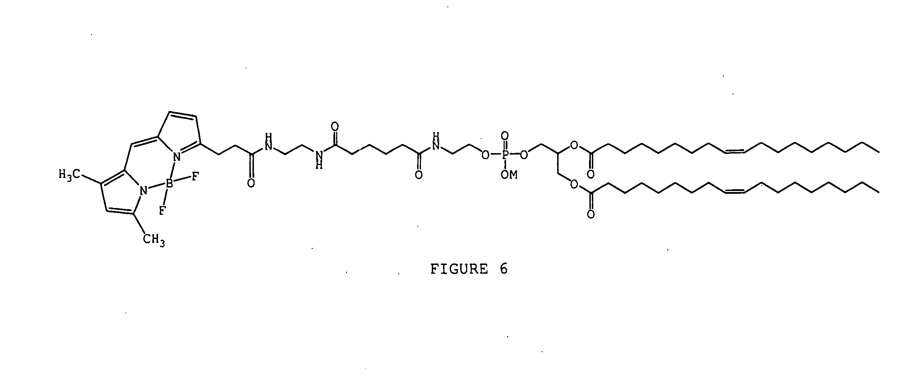

**Figure 6****.** Structure of cell marker designated KODE-BODIPY (**V**).

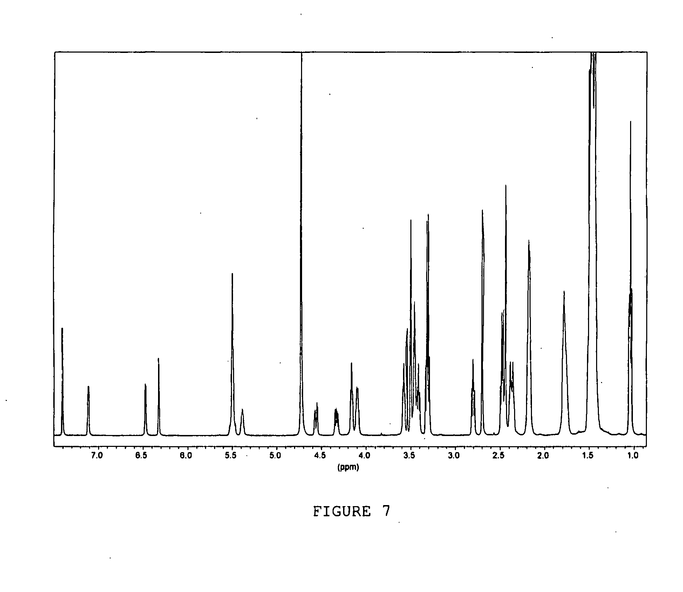

**Figure 7****.** ¹H-NMR spectrum of the cell marker designated KODE-BODIPY (**V**).

### DETAILED DESCRIPTION

The specification accompanying international application no. PCT/NZ2005/000052 (publication no. WO 2005/090368) describes water soluble synthetic molecules that are constructs of the structure F-S₁-S₂-L.

In these constructs F is a carbohydrate and the constructs spontaneously and stably incorporate into lipid bi-layers, including cell membranes.

The preferred constructs described in the specification accompanying the international application comprise the substructure: 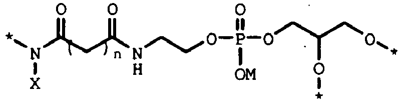 where n = 3 to 5, X is H or C, and * is other than H.

M is typically H, but may be replaced by another monovalent cation such as Na⁺, K⁺ or NH₄⁺.

F is a fluorophore in the constructs of the present invention with different physicochemical properties to those of carbohydrate. The spacer (S₁-S₂) is selected to provide a construct that can be readily dispersed in aqueous vehicles such as saline.

Whilst not wishing to be bound by theory it is believed the cell markers of the present invention spontaneously incorporate into the lipid bi-layer of the cell membrane via their diacyl lipid tail. The fluorophore moiety is therefore expressed at the cell surface. The cell markers of the present invention can be used to mark cells without modification of the proteins expressed at the surface of the cell.

The likelihood of cell functions mediated by proteins expressed at the cell surface is reduced. Furthermore, the likelihood of the cell marker becoming uniformly distributed in the two dimensions of the lipid bilayer is increased. The mobility of the fluorophore is not dependent on the mobility of the cell surface expressed proteins to which the fluorophore might otherwise be conjugated.

Additional advantages are anticipated to accrue as the cell markers may allow studies on cell membrane dynamics independent of protein function and cycling. Cells labeled using the cell markers of the present invention may still be identified by conventional means and used in established biological methods such as fluorescence activated cell sorting (FACS) systems.

For the preparation of KODE-fluorescein (**I**), FITC is first conjugated with a diamine such as 1,5-diaminopentyl (cadaverine). The conjugated FITC is then reacted with an activated lipid (L-A) prepared as described in international application number PCT/NZ2005/000052.

A number of fluorescent compounds are available commercially as cadaverine derivatives. The cell markers where F is one of the fluorophores designated in Table 1 may be prepared.

**Table 1. Fluorophores (represented as neutrally charged protonated species).**

| **Fluorophore** | **Designation** |
|---|---|
| 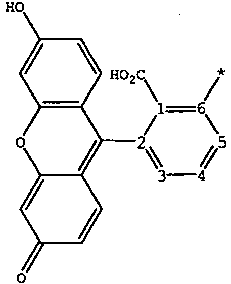 | Fluorescein (6-isomer) |
| 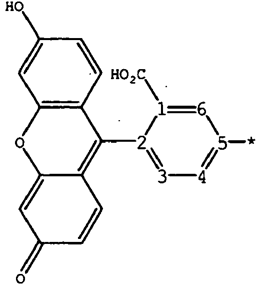 | Fluorescein (5-isomer) |
| 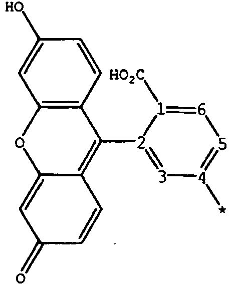 | Fluorescein (4-isomer) |
| 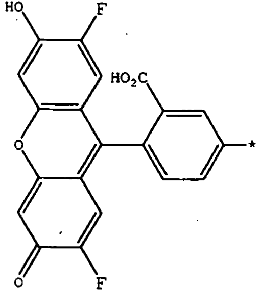 | Oregon Green (5-isomer) |
| 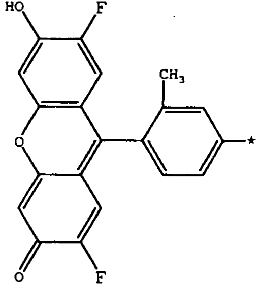 | Pennsylvania Green (5-isomer) |
| 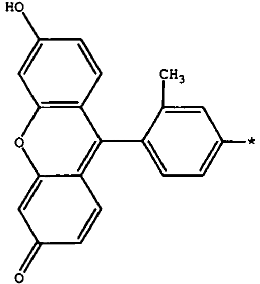 | Tokyo Green (5-isomer) |
| 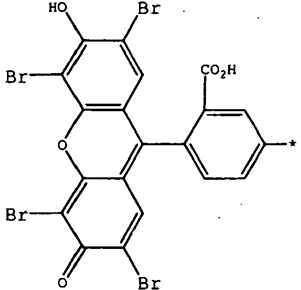 | Eosin (5-isomer) |
| 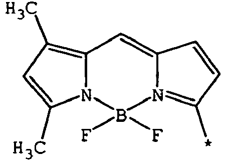 | BODIPY |
| 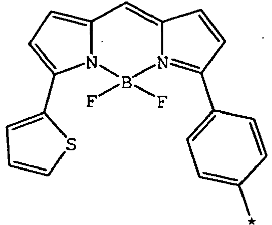 | BODIPY TR |
| 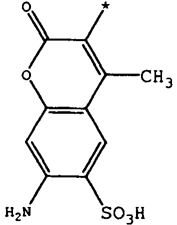 | Alexa Fluor 350 |
| 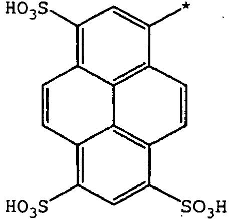 | Alexa Fluor 405 |
| 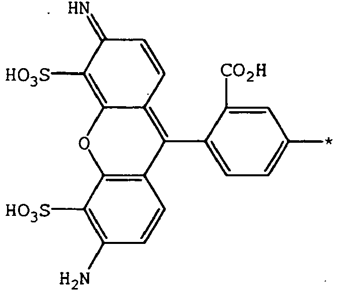 | Alexa Fluor 488 (5-isomer) |
| 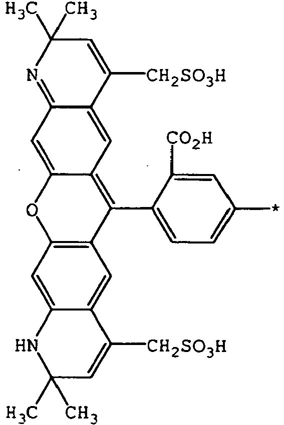 | Alexa Fluor 568 (5-isomer) |
| 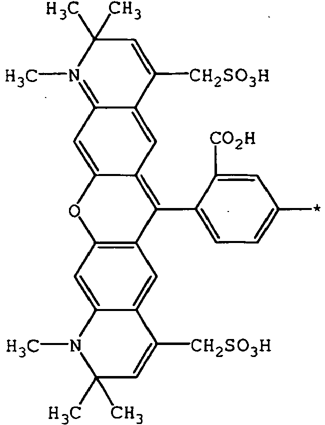 | Alexa Fluor 594 (5-isomer) |
| 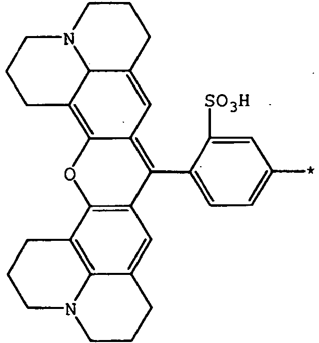 | Texas Red (5-isomer) |
| 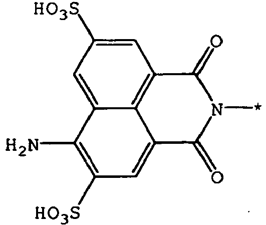 | Lucifer Yellow |
| 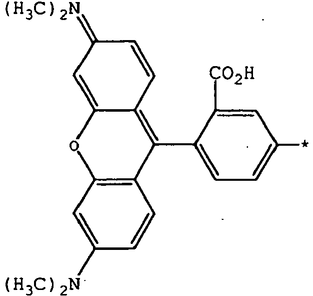 | Tetramethylrhodamine (5-isomer) |

For the preparation of KODE-BODIPY (V), BODIPY may alternatively be conjugated with an alkionyl diamine such propionly ethylenediamine (BODIPY FL EDA). The conjugated BODIPY is then reacted with an activated lipid (L-A) prepared as described in the specification accompanying international application no. PCT/NZ2005/000052.

### EXAMPLE 1

### Preparation of activated 1,2-O-distereoyl-sn-glycero-3-phosphatidylethanolamine (DSPE) and activated 1,2-O-dioleoyl-sn-glycero-3-phosphatidylethanolamine (DOPE)(L-A)

To a solution of bis(N-hydroxysuccinimidyl) adipate (A) (70 mg, 205 µmol) in dry N,N-dimethylformamide (1.5 ml) were added DOPE or DSPE (L) (40 µmol) in chloroform (1.5 ml) followed by triethylamine (7 µl). The mixture was kept for 2 h at room temperature, then neutralized with acetic acid and partially concentrated *in vacuo.*

Column chromatography (Sephadex LH-20, 1:1 chloroform-methanol, 0.2% acetic acid) of the residue yielded the activated lipid (L-A) (37 mg, 95%) as a colorless syrup; TLC (chloroform-methanol-water, 6:3:0.5): R_{f} = 0.5 (DOPE-A), R_{f} = 0.55 (DSPE-A).

¹H NMR (CDCl₃/CD₃OD, 2:1), δ:

DSPE-A - 5.39 (m, 1H, -OCH₂-C*H*O-CH₂O-), 4.53 (dd, 1H, J=3.42, J=11.98, -CCOOHC*H*-CHO-CH₂O-), 4.33 (dd, 1H, J=6.87, J=11.98, - CCOO*H*CH-CHO-CH₂O-), 4.23 (m, 2H, PO-C*H₂*-CH₂-NH₂), 4.15 (m, 2H, -C*H*₂-OP), 3,61 (m, 2H, PO-CH₂-C*H₂*-NH₂), 3.00 (s, 4H, ONSuc), 2.81 (m, 2H, -C*H*₂-CO (Ad), 2.48 (m, 4H, 2x(-C*H₂*-CO), 2.42 (m, 2H, -C*H₂*-CO (Ad), 1.93 (m, 4H, COCH₂C*H₂*C*H₂*CH₂CO), 1.78 (m, 4H, 2x(COCH₂C*H₂*-), 1,43, 1.47 (2 bs, 40H, 20CH₂), 1.04 (m, 6H, 2CH₃).

DOPE-A - 5.5 (m, 4H, 2x(-C*H*=C*H*-), 5.39 (m, 1H, -OCH₂-C*H*O-CH₂O-), 4.58 (dd, 1H, J=3.67, J=11.98, -CCOOHC*H*-CHO-CH2O-), 4.34 (dd, 1H, J=6.61, J=11.98, -CCOO*H*CH-CHO-C*H₂*O-), 4.26 (m, 2H, PO-C*H₂*-CH₂-NH₂), 4.18 (m, 2H, -C*H₂*-OP), 3.62 (m, 2H, PO-CH₂-C*H₂*-NH₂), 3.00 (s, 4H, ONSuc), 2.8 (m, 2H, -C*H₂*-CO (Ad), 2.50 (m, 4H, 2x(-C*H₂*-CO), 2.42 (m, 2H, -C*H₂*-CO (Ad), 2.17 (m, 8H, 2x(-C*H₂*-CH=CH-C*H₂*-), 1.93 (m, 4H, COCH₂C*H₂*C*H₂*CH₂CO), 1.78 (m, 4H, 2x(COCH₂C*H₂*-), 1,43, 1.47 (2 bs, 40H, 20CH₂), 1.04 (m, 6H, 2CH₃).

### Condensation of DOPE-A with 5-((5-aminopentyl)thioureidyl) fluorescein (fluorescein cadaverine)

To a solution of activated DOPE (L-A) (5 mg, 5.2 µmol) in N,N-dimethylformamide (0.5 ml) 3 mg (4.6 µmol) of fluorescein cadaverine dihydrobromide salt and 5 µl of triethylamine were added. The mixture was kept for 2 h at room temperature, then 10 µl of 3% aq. NH₃ were added and the mixture was kept at room temperature for 1 h.

Column chromatography (Sephadex LH-20, 1:1 chloroform-methanol, followed by silica gel, ethyl acetate-isopropanol-water, 6:3:1) of the mixture yielded 4.2 mg (67%) KODE-fluorescein (**I**), R_{f} 0.5 (ethyl acetate-isopropanol-water, 6:3:1).

¹H NMR (CDCl₃/CD₃OD, 1:1), δ:

KODE-fluorescein (**I**) - 8.38 (bs, 1H, aromatic proton of fluorescein), 8.15 (dd, 1H, *J*=1.7, *J*=8.3, aromatic proton of fluorescein) 7.30(d, 1H, *J*=8.3, aromatic proton of fluorescein), 6.87 (m, 4H, aromatic protons of fluorescein), 6.72 (dd, 2H, *J*=2.4, *J*=8.8, aromatic protons of fluorescein), 5.50 (m, 4H, 2×(-CH=CH-), 5,38 (m, 1H, -OCH₂ -CHO-CH₂O-), 4.58 (dd, 1H, *J*=6.6, *J_{gem}*=11.8, HHC-O-C(O)-), 4.34 (dd, 1H, *J*=3.2, *J_{gem}=*11.8, HHC-O-C(O)-), 4.14 (m, 2H, -OCH-CH₂-O-P-)(4.1 (m, 2H, -P-O-CH₂-CH₂-NH-) 3.80 (m, 2H, N-CH₂(CH₂)₃-CH₂NH-C=S) 3.39 and 3.58 (2m, 2x2H, N- CH₂-CH₂-O-P- and N-CH₂-(CH₂)₃-CH₂NH-C=S) 2.48 (m, 4H, 2×(-CH₂-CO), 2.39 (m, 4H, COCH₂CH₂CH₂CH₂CO), 2.19 (m, 8H, 2×(-CH₂-CH=CH-CH₂-), 1.84 (m, 2H, CH₂- fluorescein cadaverine), 1.8 (m, 10H, COCH₂CH₂CH₂CH₂CO, 2×(COCH₂CH₂-, and CH₂-- fluorescein cadaverine), 1.62 (m, 2H, CH₂- fluorescein cadaverine) 1,42, 1.46 (2 bs, 40H, 20 CH₂), 1.05 (m, 6H, 2 CH₃).

### Association of KODE-fluorescein (I) with cell membranes

KODE-fluorescein (**I**) readily associates with the membrane of red blood cells. Insertion of the molecule is observed when dispersions of the molecule at concentrations greater than 0.1 mg/ml are contacted with suspensions of the red blood cells.

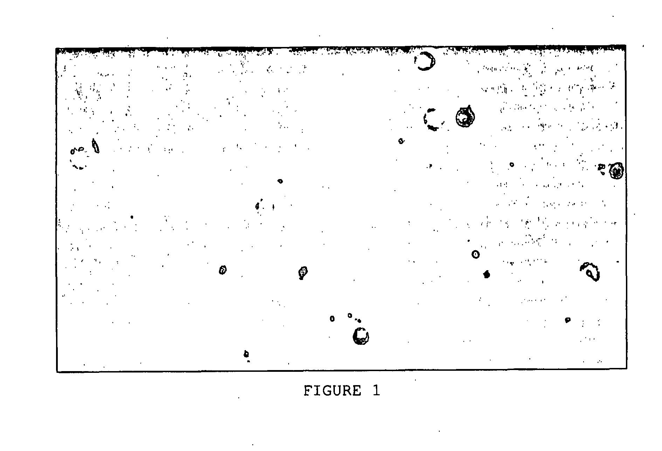

A medium to strongly fluorescing cell was considered to indicate a uniform distribution of the molecule across the cell membrane (Figure 1). The incorporation and distribution appears to be stable for a period of at least 40 days when cells are stored in the dark.

### EXAMPLE 2

Activated 1,2-O-dioleoyl-sn-glycero-3-phosphatidylethanolamine (DOPE)(L-A) was prepared as decribed in Example 1.

### Condensation of DOPE-A with 4,4-difluoro-5,7-dimethyl-4-bora-3a,4a-diaza-s-indacene-3-propionyl ethylenediamine, hydrochloride (BODIPY FL EDA)

To a solution of 15 mg (15.5 µmol) activated DOPE in CH₂Cl₂ (0.5 ml), 5 mg (13.5 µmol) of BODIPY FL EDA in N,N-dimethylformamide (0.3 ml) and 5 µl of triethylamine were added. The mixture was kept for 2 h at room temperature.

Column chromatography (Sephadex LH-20, 1:1 chloroform-methanol) of the mixture yielded 14.2 mg (75%) KODE-BODIPY (**I**), Et₃N-salt; MW 1289.6, R_{f} 0.3 (ethyl acetate-isopropanol-water, 6:3:1).

¹H NMR (CDCl_{3/}CD₃OD, 1:1): δ 7.40 (s, 1H, aromatic proton of BODIPY), 7.12 (d, 1H, *J*=3.8 aromatic proton of BODIPY), 6.47 (d, 1H, *J*=3.8 aromatic proton of BODIPY), 6.32 (s, 1H, aromatic protons of BODIPY), 5.50 (m, 4H, 2x(-CH=CH-), 5.38 (m, 1H, -OCH₂-CHO-CH₂O-), 4.58 (dd, 1H, *J*=3.2, *J_{gem}*=11.8, HHC-O-C(O)-), 4.33 (dd, 1H, J=6.6, *J_{gem}*=11.8, HHC-O-C(O)-), 4.16 (t, 2H, *J*=5.6, P-O-CH₂-CH₂-NH-), 4.1 (m, 2H, - -OCH-CH₂-O-P-), 3.60 (t, 2H, P-O-CH₂-CH₂-NH-), 3.46, 3.42 and 2.8 (3m, 4H, 2H, 2H, -CH₂-CH₂-C(O)NH(CH₂)₂-NH of BODIPY), 2.70 (s, 3H, CH₃ of BODIPY), 2.48 (m, 4H, 2x(-CH₂-CO), 2.45 (s, 3H, CH₃ of BODIPY), 2.37 (m, 4H, COCH₂CH₂CH₂CH₂CO), 2.19 (m, 8H, 2x(-CH₂-CH=CH-CH₂-), 1.8 (m, 8H, COCH₂CH₂CH₂CH₂CO, 2x(COCH₂CH₂-)), 1.46, 1.43 (2 bs, 40H, 20 CH₂), 1.05 (m, 6H, 2 CH₃) ; 3.31 (q, 6H, J=7.4, 3xCH₂ of Et₃N), 1.50 (t, 9H, J=7.4, 3xCH₃ of Et₃N).

Although the invention has been described by way of exemplary embodiments it should be appreciated that variations and modifications may be made with out departing from the scope of the invention.

## Claims

1. A **fluorescent cell marker** of the structure:
F-S₁-S₂-L
including the substructure: 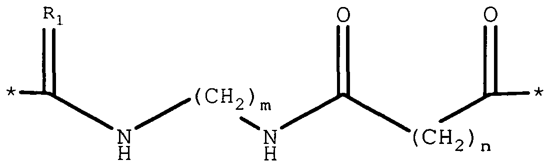 where:
F is a fluorophore;
S₁-S₂ is a spacer linking F to L;
L is a lipid selected from the group consisting of diacyl- and dialkyl-glycerolipids, including glycerophospholipids;
m and n are independently 3 to 6;
R₁ is O or S; and
* is other than H.

2. The fluorescent cell marker of claim 1 where F is selected from the group consisting of: fluorophores of fluorescein, Oregon Green, Pennsylvania Green, Tokyo Green, eosin, BODIPY, BODIPY TR, Alexa Fluor 350, Alexa Fluor 405, Alexa Fluor 488, Alexa Fluor 568, Alexa Fluor 594, Texas Red, Lucifer Yellow, tetramethylrhodamine and their derivatives, preferably where F is selected from the group consisting of:
fluorophores of fluorescein, BODIPY and their derivatives.

3. The fluorescent cell marker of claim 1 or 2 where the sum of m and n is 6 to 9 and * is C or N.

4. The fluorescent cell marker of any one of claims 1 to 3 where F is the fluorophore of fluorescein or one of its derivatives and S₁ is a C₃₋₅-diaminoalkyl derivative selected from the group consisting of: 1,3-diaminopropyl, 1,4-diaminobutyl, or 1,5-diaminopentyl derivatives, preferably S₁ is a C₃₋₅-aminoalkylthioureidyl, preferably S₁ is 5-((5-aminopentyl) thioureidyl.

5. The fluorescent cell marker of any one of claims 1 to 4 where F is the fluorophore of fluorescein or one of its derivatives and S₂ is selected from the group consisting of: -CO(CH₂)₃CO-, -CO(CH₂)₄CO- (adipate), and -CO(CH₂)₅CO-, preferably -CO(CH₂)₄CO- (adipate).

6. The fluorescent cell marker of any one of claims 1 to 5 where F is the fluorophore of fluorescein or one of its derivatives and the structure includes the substructure: 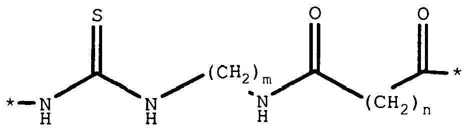 where m and n are independently 3 to 5 and * is other than H.

7. The fluorescent cell marker of any one of claims 1 to 3 where F is the fluorophore of BODIPY or one of its derivatives and S₁ is a C₃₋₅-alkionyldiamine, preferably S₁ is propionyl ethyldiamine.

8. The fluorescent cell marker of any one of claims 1 to 3 or 7 where F is the fluorophore of BODIPY or one of its derivatives and S₂ is selected from the group consisting of: -CO(CH₂)₃CO-, -CO(CH₂)₄CO- (adipate) and -CO(CH₂)₅CO-, preferably S₂ is -CO(CH₂)₄CO- (adipate).

9. The fluorescent cell marker of any one of claims 1 to 3, 7 or 8 where F is the fluorophore of BODIPY or one of its derivatives and the structure includes the substructure: 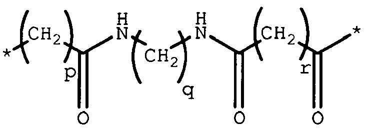 where p, q and r are independently 3 to 5 and * is other than H, preferably where the sum of p, q and r is 8, preferably where p is 2, q is 2 and r is 4.

10. The fluorescent cell marker of any one of claims 1 to 9 where L is selected from the group consisting of: diacylglycerolipids, phosphatidate, phosphatidyl choline, phosphatidyl ethanolamine, phosphatidyl serine, phosphatidyl inositol, phosphatidyl glycerol, and diphosphatidyl glycerol derived from one or more of *trans*-3-hexadecenoic acid, *cis*-5-hexadecenoic acid, cis-7-hexadecenoic acid, *cis*-9-hexadecenoic acid, *cis-*6-octadecenoic acid, *cis*-9-octadecenoic acid, *trans*-9-octadecenoic acid, *trans*-11-octadecenoic acid, *cis*-11-octadecenoic acid, *cis*-11-eicosenoic acid or *cis*-13-docsenoic acid, preferably the lipid is derived from one or more *cis*-desaturated fatty acids, more preferably L is selected from the group consisting of: 1,2-*O*-dioleoyl-*sn*-glycero-3-phosphatidylethanolamine (DOPE) and 1,2-*O*-distearyl-*sn*-glycero-3-phosphatidylethanolamine (DSPE).

11. A **fluorescent cell marker** with the structure: 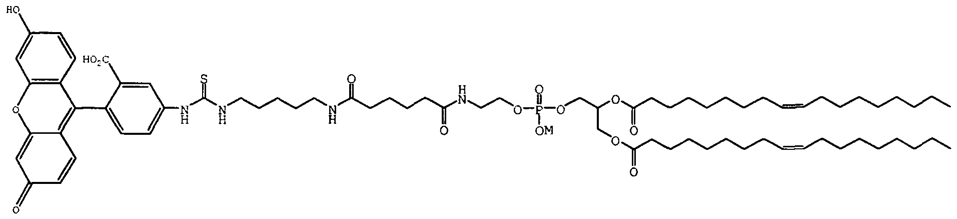 or 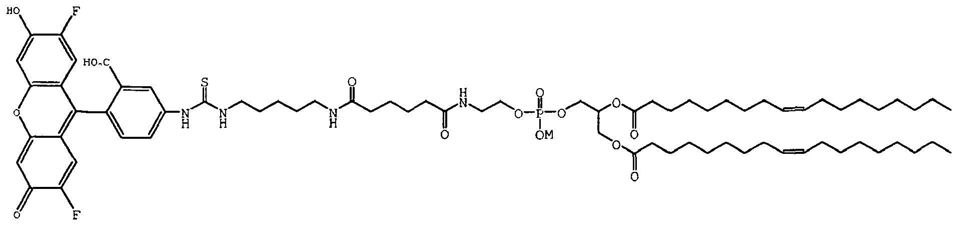 or 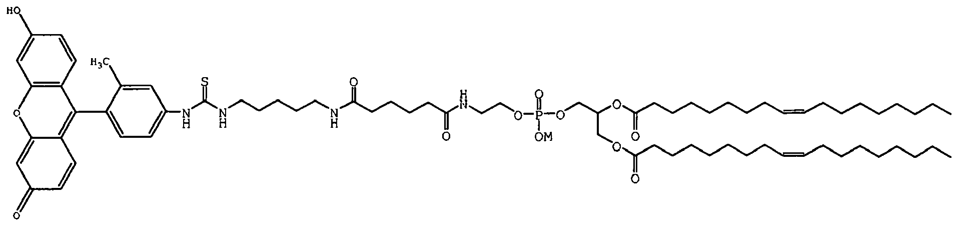 or 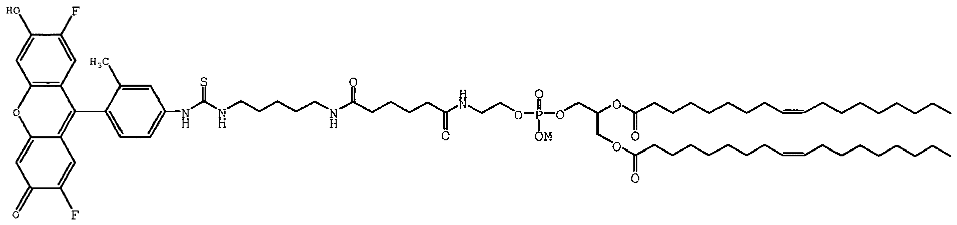 or 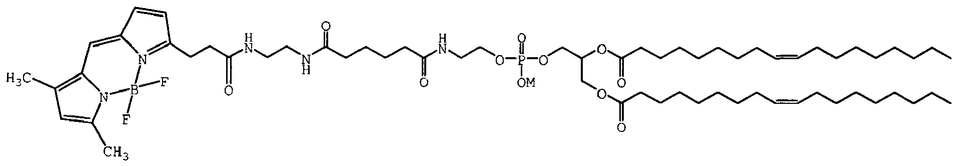 where M is a monovalent cation, preferably H⁺, Na⁺, K⁺ or NH₄⁺, more preferably H⁺.

12. A **method** of marking cells including the step of:
• Contacting a suspension of cells with a cell marker of any preceding claim for a time and at a temperature sufficient to allow incorporation of the marker into the membrane of the cell.

13. A **cell** incorporating a cell marker of any one of claims 1 to 11.

## Patentansprüche

1. **Fluoreszierender Zellenmarker** mit der Struktur: F-S₁-S₂-L
mit der Substruktur: 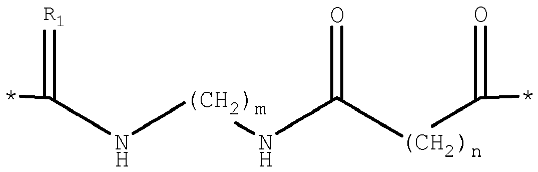
worin:
F für ein Fluorophor steht;
S₁-S₂ für einen Abstandhalter steht, der F mit L verbindet;
L für ein Lipid steht, das aus der Gruppe ausgewählt ist, die aus Diacyl- und Dialkylglycerolipiden, einschließlich Glycerophospholipiden, besteht;
m und n unabhängig für 3 bis 6 stehen;
R₁ für O oder S steht; und
* für etwas anderes als H steht.

2. Fluoreszierender Zellmarker nach Anspruch 1, worin F aus der Gruppe ausgewählt ist, die aus Folgendem besteht: Fluorophore von Fluorescein, Oregon Green, Pennsylvania Green, Tokyo Green, Eosin, BODIPY, BODIPY TR, Alexa Fluor 350, Alexa Fluor 405, Alexa Fluor 488, Alexa Fluor 568, Alexa Fluor 594, Texas Red, Lucifer Yellow, Tetramethylrhodamin und deren Derivate, worin F bevorzugt aus der Gruppe ausgewählt ist, die aus: Fluorophoren von Fluorescein, BODIPY und ihren Derivaten besteht.

3. Fluoreszierender Zellmarker nach Anspruch 1 oder 2, worin die Summe von m und n 6 bis 9 beträgt und * für C oder N steht.

4. Fluoreszierender Zellmarker nach irgendeinem der Ansprüche 1 bis 3, worin F für das Fluorophor von Fluorescein oder eines seiner Derivate steht und S₁ für ein C₃₋₅-Diaminoalkylderivat steht, das aus der Gruppe ausgewählt ist, die aus: 1,3-Diaminopropyl, 1,4-Diaminobutyl oder 1,5-Diaminopentylderivaten besteht, vorzugsweise steht S₁ für ein C₃₋₅-Aminoalkylthioureidyl, bevorzugt steht S₁ für 5-((5-Aminopentyl)-thioureidyl.

5. Fluoreszierender Zellmarker nach irgendeinem der Ansprüche 1 bis 4, worin F für das Fluorophor von Fluorecein oder eines seiner Derivate steht und S₂ aus der Gruppe ausgewählt ist, die aus: -CO(CH₂)₃CO-, -CO(CH₂)₄CO-(adipat) und -CO(CH₂)₅CO-, bevorzugt -CO(CH₂)₄CO-(adipat), besteht.

6. Fluoreszierender Zellmarker nach irgendeinem der Ansprüche 1 bis 5, worin F für das Fluorophor von Fluorescein oder eines seiner Derivate steht und die Struktur die Substruktur: 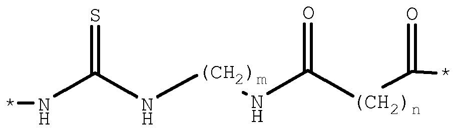 einschließt, worin m und n unabhängig für 3 bis 5 stehen und * etwas anderes als H ist.

7. Fluoreszierender Zellmarker nach irgendeinem der Ansprüche 1 bis 3, worin F für das Fluorophor BODIPY oder eines seiner Derivate steht und S₁ für ein C₃₋₅-Alkionyldiamin steht, bevorzugt steht S₁ für Propionylethyldiamin.

8. Fluoreszierender Zellmarker nach irgendeinem der Ansprüche 1 bis 3 oder 7, worin F für das Fluorophor von BODIPY oder eines seiner Derivate steht und S₂ aus der Gruppe ausgewählt ist, die aus: -CO(CH₂)₃CO-, -CO(CH₂)₄CO-(adipat) und -CO(CH₂)₅CO- steht, bevorzugt steht S₂ für -CO(CH₂)₄CO-(adipat).

9. Fluoreszierender Zellmarker nach irgendeinem der Ansprüche 1 bis 3, 7 oder 8, worin F für das Fluorophor von BODIPY oder eines seiner Derivate steht und die Struktur die Substruktur: 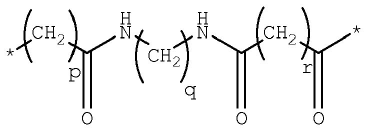 einschließt, worin p, q und r unabhängig für 3 bis 5 stehen und * etwas anders als H ist, worin vorzugsweise die Summe von p, q und r 8 beträgt, worin vorzugsweise p für 2, q für 2 und r für 4 steht.

10. Fluoreszierender Zellmarker nach irgendeinem der Ansprüche 1 bis 9, worin L aus der Gruppe ausgewählt ist, die aus Folgendem besteht: Diacylglycerolipide, Phosphatidat, Phosphatidylcholin, Phosphatidylethanolamin, Phosphatidylserin, Phosphatidylinositol, Phosphatidylglycerol und Diphosphatidylglycerol, das von einer oder mehreren von *trans*-3-Hexadecensäure, *cis*-5-Hexadecensäure, *cis*-7-Hexadecensäure, *cis*-9-Hexadecensäure, *cis*-6-Octadecensäure, *cis*-9-Octadecensäure, *trans*-9-Octadecensäure, *trans*-11-Octadecensäure, *cis*-11-Octadecensäure, *cis*-11-Eicosensäure oder *cis*-13-Docsensäure abgeleitet ist, vorzugsweise ist das Lipid von einer der mehreren *cis*-entsättigten Fettsäuren abgeleitet, mehr bevorzugt ist L aus der Gruppe ausgewählt, die aus: 1,2-*O*-Dioleoyl-*sn*-glycero-3-phosphatidylethanolamin (DOPE) und 1,2-O-Distearyl-*sn*-glycero-3-phosphatidylethanolamin (DSPE) besteht.

11. **Fluoreszierender Zellmarker** mit der Struktur: 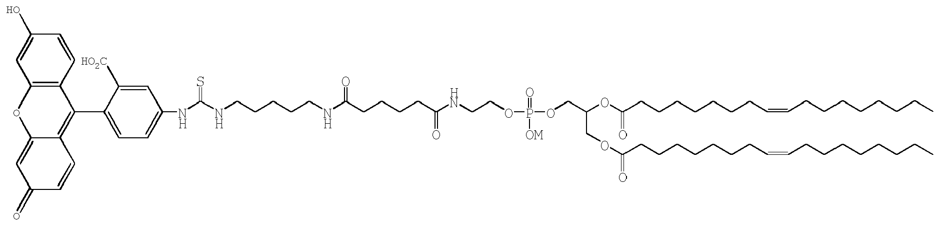 oder 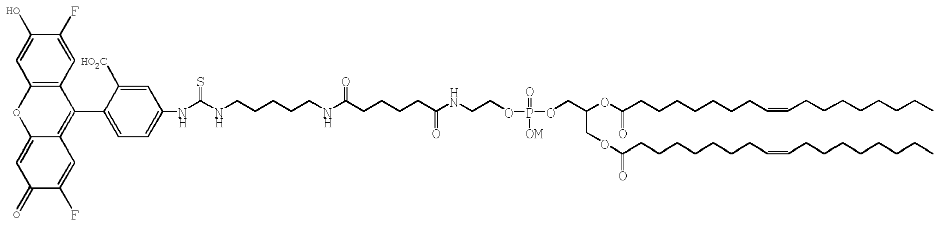 oder 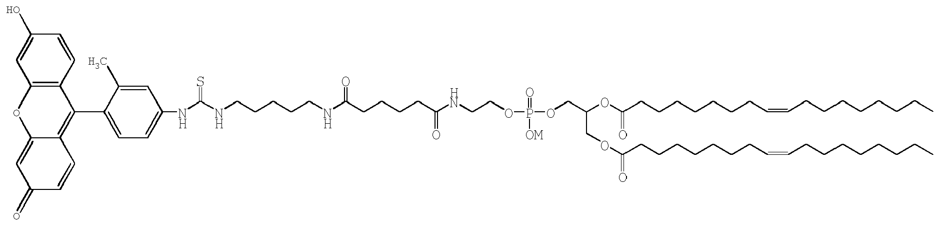 oder 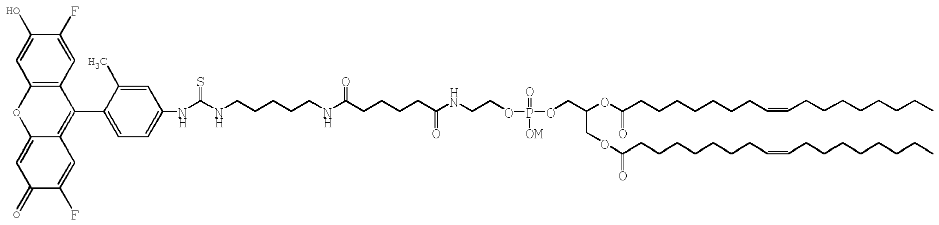 oder 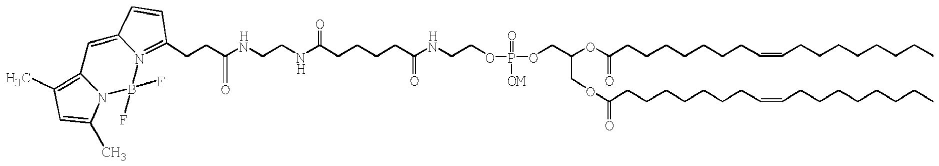 worin M für ein monovalentes Kation, bevorzugt H⁺, Na⁺, K⁺ oder NH₄⁺, mehr bevorzugt H⁺, steht.

12. **Verfahren** zum Markieren von Zellen, das folgenden Schritt einschließt:
• Inkontaktbringen einer Zellsuspension mit einem Zellmarker nach irgendeinem vorhergehenden Anspruch für eine Zeit und bei einer Temperatur, die ausreichend ist, um die Einbringung des Markers in die Membran der Zelle zu erlauben.

13. **Zelle**, die einen Zellmarker nach irgendeinem der Ansprüche 1 bis 11 beinhaltet.

## Revendications

1. Un **marqueur cellulaire fluorescent** de structure :
F-S₁-S₂-L
incluant la sous-structure : 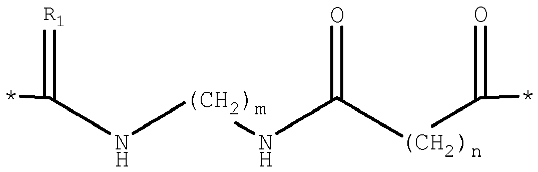 où :
F représente un fluorophore ;
S₁-S₂ est un espaceur de liaison F à L, et
L est un lipide choisi dans le groupe constitué de diacyl- et dialkyl-glycérolipides, y compris les glycérophospholipides, et
m et n sont indépendamment 3-6, et
R₁ est 0 ou S, et * est différent de H.

2. Le marqueur cellulaire fluorescent selon la revendication 1, où F est sélectionné dans le groupe constitué de : fluorophores de fluorescéine, Oregon Green, Pennsylvania Green, Tokyo Green, éosine, BODIPY, BODIPY TR, Alexa Fluor 350, Alexa Fluor 405, Alexa Fluor 488, Alexa Fluor 568, Alexa Fluor 594, Texas Red, Lucifer Yellow, tétraméthylrhodamine et leurs dérivés, et de préférence, où F est sélectionné dans le groupe constitué de : fluorophores de fluorescéine, BODIPY et leurs dérivés.

3. Le marqueur cellulaire fluorescent de la revendication 1 ou 2 où la somme de m et n est 6 à 9, et * est C ou N.

4. Le marqueur cellulaire fluorescent selon une quelconque des revendications 1 à 3 où F est le fluorophore de fluorescéine ou l'un de ses dérivés, et S₁ est un dérivé C₃₋₅-diaminoalkyl sélectionné dans le groupe constitué de : 1,3-diaminopropyl, 1,4-diaminobutyl, ou les dérivés de 1,5-diaminopentyl, S₁ est, de préférence, un C₃₋₅-aminoalkylthioureidyl, et S₁ est, de préférence, 5-((5-aminopentyl) thioureidyl.

5. Le marqueur cellulaire fluorescent selon l'une quelconque des revendications 1 à 4 où F est le fluorophore de fluorescéine ou l'un des ses dérivés et S₂ est sélectionné dans le groupe constitué de : - CO(CH₂)₃CO-, -CO(CH₂)₄CO- (adipate), et -CO(CH₂)₅CO-, de préférence -CO(CH₂)₄CO-(adipate).

6. Le marqueur cellulaire fluorescent selon l'une quelconque des revendications 1 à 5 où F est le fluorophore de fluorescéine ou l'un des ses dérivés et la structure inclut la sous-structure : 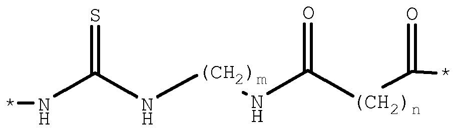 où m et n sont indépendamment 3-5 et * est différent de H.

7. Le marqueur cellulaire fluorescent selon l'une quelconque des revendications 1 à 3 où F est le fluorophore de BODIPY ou l'un de ses dérivés et S₁ est un C₃₋₅-alkionyldiamine et S₁ est, de préférence, éthyldiamine proprionyle.

8. Le marqueur cellulaire fluorescent selon l'une quelconque des revendications 1 à 3 ou 7, où F est le fluorophore de BOPIDY ou l'un des ses dérivés et S₂ est sélectionné dans le groupe constitué de : - CO(CH₂)₃CO-, -CO(CH₂)₄CO-(adipate) et -CO(CH₂)₅CO-, et S₂ est, de préférence, -CO(CH₂)₄CO-(adipate).

9. Le marqueur cellulaire fluorescent selon l'une quelconque des revendications 1 à 3, 7, ou 8, où F est le fluorophore de BOPIDY ou l'un des ses dérivés et la structure inclut la sous-structure : 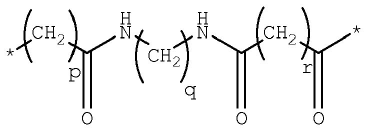 où p, q et r sont indépendamment 3-5 et * est différent de H, et de préférence où la somme de p, q et r est 8, et encore de préférence, où p est 2, q est 2 et r est 4.

10. Le marqueur cellulaire fluorescent selon l'une quelconque des revendications 1 à 9, où L est sélectionné dans le groupe constitué de :
diacylglycérolipides, phosphatidate, phosphatidyl choline, phosphatidyl éthanolamine, phosphatidyl sérine, phosphatidyl-inositol, phosphatidyl-glycérol, et diphosphatidylglycérol dérivé d'un ou plusieurs parmi l'acide *trans*-3-hexadécénoïque, l'acide *cis*-5-hexadécénoïque, l'acide *cis*-7-hexadécénoïque, l'acide *cis*-9-hexadécénoïque, l'acide *cis*-6-octadécénoïque,
l'acide *cis*-9-octadécénoïque, l'acide *trans*-9-octadécénoïque, l'acide *trans*-11-octadécénoïque,
l'acide *cis*-11-octadécénoïque, l'acide *cis*-11-éicosénoïque ou l'acide *cis*-13-docsénoïque, et où le lipide est dérivé, de préférence, d'un ou plusieurs parmi les acides gras *cis*-désaturés, et de manière plus préférentielle, où L est sélectionné dans le groupe constitué de : 1,2-O-dioléoyl-sn-glycéro-3-phosphatidyléthanolamine (DOPE) et 1,2-O-distéaryl-*sn-*glycéro-3-phosphatidyléthanolamine (DSPE).

11. Un **marqueur cellulaire fluorescent** de structure : 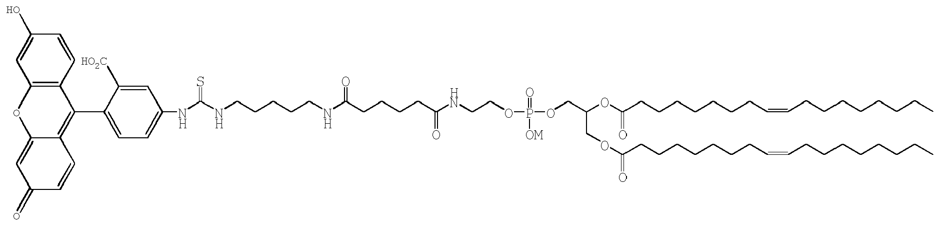 ou 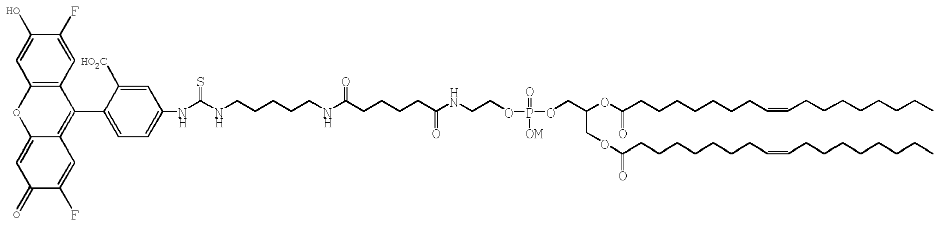 ou 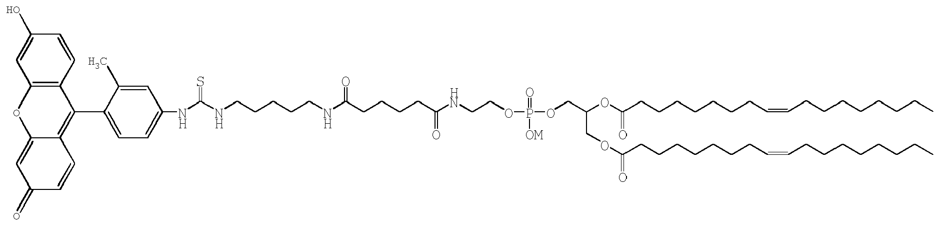 ou 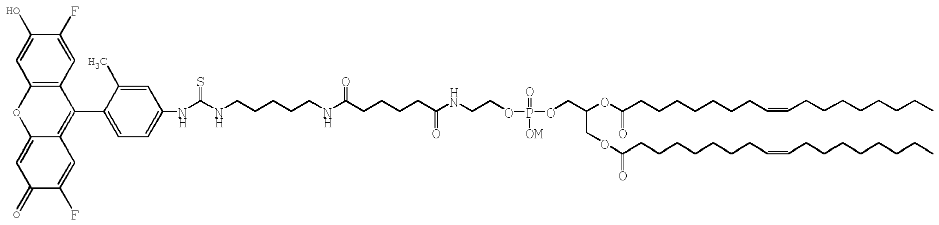 ou 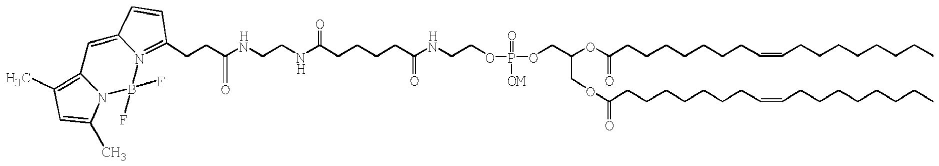 où M est un cation monovalent, de préférence H⁺, Na⁺, K⁺ ou NH₄⁺, et de manière plus préférentielle, H⁺.

12. Un **procédé** de marquage cellulaire comprenant l'étape suivante :
• Contact entre une suspension de cellules et un marqueur cellulaire selon l'une quelconque des revendications précédentes sur une durée et à une température suffisantes pour permettre l'incorporation du marqueur dans la membrane cellulaire.

13. Une **cellule** incorporant un marqueur cellulaire selon l'une quelconque des revendications 1 à 11.
